# EUROPEAN PATENT APPLICATION

(11) **EP 3 381 491 A1**
(43) Date of publication of application: **03.10.2018**
(21) Application number: 17163423.1
(22) Date of filing: 28.03.2017
(51) Int. Cl.: A61M 5/24, A61M 5/32

(54) **PEN CAP FOR AN INJECTION DEVICE COMPRISING A NEEDLE DESTROYER**

(71) Applicant: Tecpharma Licensing AG, 3401 Burgdorf (CH)
(72) Inventor: BURREN, Stefan, 3150 Schwarzenburg (CH); MORI, Kevin, 3400 Burgdorf (CH); STEINER, Fabian, 3400 Burgdorf (CH)

(57) **Abstract**

Injection pen comprising a pen cap (1) for enclosing a cartridge holder (2) of the injection pen whereby the pen cap comprises a cylindrical wall section (1c) connecting an opening (1e) at the proximal end for inserting the cartridge holder (2) with a distal end wall (1 b) for closing the pen cap. The pen cap comprises furthermore a cylindrical wall section (1c) made from a first material (11) and the cap is characterized in that the distal end wall (1b) comprises a second material (12) reinforcing the distal end wall (1b) and preventing needle stitching through the distal end wall (1b).

## Description

### Background

Injection devices are used for the subcutaneous delivery of liquid medicaments to a patient. Such injection devices are often pen-shaped, having a long axis and are called injection pens. The injection pens comprise a housing, which can hold a dose setting and dose delivery mechanism. The medication is preferably present in a cartridge or in a prefilled syringe. A cartridge is normally attached to the housing of the injection pen using a cartridge holder and a prefilled syringe is held by a syringe holder that is engaged directly or indirectly with the housing. The injection pen comprises a pen cap enclosing the cartridge holder when the pen cap is attached to the injection pen.

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

Such cartridges are preferably made from glass and have a cylindrical shape, which is narrowed at one end and closed by a pierceable septum. The open end of the cylinder is closed off with a sealing plug after having been filled with the liquid medication. The liquid medication is thus present between the septum and the plug. The cartridge is attached to the housing, or to a housing part using a cartridge holder. The cartridge holder has a cylindrical shape which is open on one side to receive the cartridge and which has a narrow section having a connector for connecting a needle to receive the cartridge holder. The needle has one sharp end for penetrating the septum and the opposite end of the needle, which is sharpened as well, is intended for piercing the skin of a patient. Such needles are held in a needle hub, which has the correct dimensions to fit onto the connector of the cartridge holder to properly align the needle for piercing the septum of the cartridge and to hold the needle hub to the cartridge holder. The cartridge holder and the connector of the cartridge holder are preferably cylindrically shaped, with a central axis that coincides with the central axis of the injection pen.

The injection needles of the prior art comprise a needle and the needle hub as described previously. Additionally the distal end of the needle is first covered by a needle tip cap and subsequently the injection needle is enclosed by a needle cap. A user mounts this needle assembly by grabbing the needle cap, which enables the user to safely position the needle hub of the assembly onto the connector of the cartridge holder. Thereby, the connection between the proximal end of the needle and the contents of the cartridge is established since the proximal end pierces the septum of the cartridge. The user first removes the needle cap from the assembly and after removing the needle cap, the needle tip cap can be removed such that the distal end of the needle is available for piercing the skin of the patient. After the injection, the single use needle needs to be removed and the user normally re-attaches the needle cap to the needle to safely remove the assembly without the risk of needle stitching.

A pen cap is located at the distal end of the injection pen and the pen cap is preferably attached or attachable to the housing or housing part. The pen cap preferably encloses the cartridge holder and is removed before attaching the needle to the cartridge holder and intended to be replaced on the pen after the single use needle has been discarded.

In EP 0498737, a pen cap is shown which, once attached to the injection pen, leaves sufficiently headspace within the cap to enclose a needle hub with a needle and a needle tip cap. The patient can leave a needle that has been used once onto the cartridge holder, therefore there is contact between the ambient and the medicament after the injection which can reduce the quality of the medicament, for example due to oxidative degradation. Moreover, the user is not aware that a used needle is still on the device, therewith increasing the risk of multiple use of a needle that is intended for single use only.

In US 6761705, a pen cap for an injection pen is described having an end surface that can pivot in front of the needle tip of the needle. The pen cap and end surface are made of metal and once the pen cap is closed, the tip of the needle is destroyed by the end surface.

In US2008108951, a pen cap comprising a compartment for storing a needle unit is disclosed. The cap is divided in a dust sleeve and a storage compartment. The dust sleeve is closed by an end wall and the length of the dust sleeve is such that this end wall interferes with the tip of the needle when the dust sleeve is mounted onto the pen with an attached needle to the cartridge holder. No special precautions or materials are disclosed to prevent stitching of the needle tip through the end wall of the dust sleeve, and therefore the user is not protected when a needle stitches through the end wall of the dust sleeve when opening the storage compartment.

In US2005148933, a needle destroyer device is disclosed that fits over the injection pen. The device has a metal insert on the inside of the destroyer device that is intended to deflect the tip of the needle. This is a device that is separate from the pen cap of the injection device.

In US 9440031, a pen cap is disclosed having a protruding element on the inside which prevents the attachment of the cap when a needle is attached to the cartridge holder. In this case the needle is not destroyed and available for second use. The solution presented requires a certain length of the protruding element in the cap to ensure that different needle lengths (for example 12 mm) are enclosed by the pen cap without interfering with the tip of the needle. This lengthens the cap to ensure that all needles (having different lengths from 4 to 12 mm) are compatible with a single pen cap.

In the prior art pen caps, either complex solutions with pivoting parts are disclosed which are completely made of a single and expensive material (metal) or the destruction of the needle requires a separate device. Alternatively, the mounting of the pen cap onto the injection device is prevented without destroying the needle having the risk of re-using a single-use needle.

It is an object of the present invention to solve the problems of the prior art and provide a pen cap for an injection pen or an injection device which destroys the needle when mounting the cap when a needle is attached to the cartridge holder without stitching though the end-wall of the cap. Therewith the stitching risk for the user of the device is reduced. The needle destroyer provides a simple and cost-efficient solution in using low cost materials for the pen cap and locally strengthen the end wall of the cap with a stitch resistant material. It is a further object of the invention to provide a short pen cap that can be used in combination with longer needles whereby the pen cap has a means that prevents the mounting of the cap when a needle is attached to the cartridge holder. The pen cap with the means to prevent attachment to the pen can be constructed shorter, for the shorter needle lengths, for example below 8 mm. The pen cap cannot be mounted onto the device without destruction of the needle whereas for the longer needle lengths, for example above 8mm the interference of the needle with the end wall does not result in needle stitching through the end wall.

### General description

A pen cap for an injection pen is presented whereby the injection pen comprises a housing or a housing part and a cartridge holder. The cartridge holder is connected or connectable to the distal end of the housing or the housing part. The cartridge holder is designed for holding a cartridge containing a medication and attaching the cartridge to the injection pen. The injection pen has a drive mechanism for advancing a piston rod in the distal direction or dose delivery direction. The advancing piston rod advances a plug present in the cartridge for dose delivery. The cartridge holder has a second connector present at the distal end for connecting a needle hub of an injection needle to the cartridge holder. The second connector can, preferably be shaped as a screwthread present on the outside of the cartridge holder or on a narrowed part of the cartridge holder. The second connector may be designed according to a standard such as ISO 11608-2. Alternatively, the second connector is shaped as circumferential ribs surrounding the narrowed section of the cartridge holder for a snap-fit connection of the needle hub. The needle hub has a cylindrical shape that is open on one side for connecting to the second connector and is closed on the opposite side by an end wall which also holds the needle of the needle hub. The needle hub is preferably made from a polymeric material and preferably coaxially surrounds the needle which is preferably a steel needle or cannula. The needle is preferably a hollow needle passing through the needle hub and having a sharp distal tip for penetrating the skin of a patient and a sharp proximal tip for penetrating a septum of the cartridge. The needle hub with the needle can be attached to the second connector of the cartridge holder by screwing, clicking or snap-fitting the needle hub onto the second connector. The pen cap is cylindrical shaped with one open side at the proximal end and closed by a wall at the distal end. On the outside of the pen cap, preferably a clip is arranged for holding the pen.

The pen cap comprises a first connector at the proximal end of the pen cap for connecting to a counter-connector present at the distal end of the housing or housing part or at the proximal end of the cartridge holder of the injection pen. For example, the first connector can be shaped as a rim with an increased wall thickness at the proximal end of the cap or as at least one protrusion present on at least one flexible arm or a cut-out present at the proximal end of the pen cap. Alternatively, a circumferential recess at the inside of the pen cap at the proximal end forms the first connector. The protrusion or recess can be also designed as a discontinuous rim or recess.

The counter-connector can be, for example, a rim, protrusion, recess and the like that matches the first connector of the pen cap to form a releasable connection. Preferably, the connection between the pen cap and the housing is established by a pure axial movement, but alternatively a combined rotational and/or axial movement can secure the connection between the pen cap and the injection pen. Said first connector preferably forms a releasable connection of the pen cap to the injection pen, e.g., the pen cap can be attached and removed from the device multiple times.

The pen cap and housing or housing part are preferably aligned along their central axis and moved along the longitudinal axis towards each other from a first position where the first connector and counter-connector are not connected, thus leaving an axial gap or distance between the first connector and the counter connector, to a second position establishing the connection between the first connector and the counter-connector. The connection is preferably established as a snap-fit connection and once the pen cap is attached to the injection pen, the cartridge holder is fully enclosed and preferably cannot be viewed by the eye. In the second position there is preferably no, or only a minimal axial gap between the pen cap and the housing. The connection between the pen cap and the injection pen can be released by axially moving the pen cap in the distal direction and the snap-fit connection is opened, whereby the pen cap is moved from the second position towards the first position.

The pen cap is characterized by that the pen cap has means for preventing the formation of the releasable connection of the pen cap to the injection pen when the needle hub of the injection needle is connected to the second connector of the cartridge holder. The releasable connection between the pen cap having the first connector and the counter connector at the housing cannot be established, e.g. the means ensure that the pen cap cannot be moved fully from the first position to the second position. Thus an axial gap exists between the pen cap and the housing and/or a part of the cartridge holder can be seen by the eye. Seen by the eye implies that this is an undesired observation of the cartridge holder, of course, such a pen cap may comprise a viewing window for deliberately viewing the cartridge holder. The non-desired visual observation is thus located at the distal end of the housing and may be accompanied by a visual sign present in the gap and printed on the housing or cartridge holder. If the counter connector for the first connector is present at the proximal section of the cartridge holder, a small circumferential rim or stop rim may be permanently observable by the eye.

A pen cap assembly for an injection pen which comprises a pen cap for enclosing a cartridge holder present at the distal end of the injection pen. The pen cap having a first connector at the proximal end of the cap for connecting to the distal end of a housing or housing part of the injection device to secure a releasable connection of the pen cap to the injection device. The cartridge holder encloses a cartridge containing a medicament which has a second connector at the distal end for connecting a needle hub of an injection needle to the cartridge holder. The pen cap has means for preventing the formation of the releasable connection of the first connector to the distal end of the housing or housing part when the needle hub of the injection needle is connected to the second connector of the cartridge holder.

The pen cap has a hollow cylindrical shape and a distal end-wall closing the pen cap, the distal end-wall being axially arranged opposite to the opening of the pen cap comprising the first connector. The length of the cap is chosen such that the open end can fit around the cartridge holder. The length can be tuned such that either there is no head space for a needle assembly within the cap or there is sufficient head space for a needle assembly in the cap. The length of the cap can also be chosen such that only needles with a certain length properly fit within the cap. For example, shorter needles with a length of 4-8 mm do fit within the head space of the cap after being connected to the housing whereas a longer 10mm or 12 mm needle does not fit within the head space.

The means for preventing the formation of the releasable connection of the pen cap to the injection pen is a means to locally reduce the inner diameter of the hollow cylindrical shaped pen cap, such as a cylinder forming a bore-hole positioned on the inside of the pen cap at the distal end-wall. Alternatively at least one fin protrudes from the cylinder wall of the pen cap towards the central axis of the pen cap. Such a fin can start at the end-wall of the cap and end at the proximal end of the pen cap just before the connecting side starts, or alternatively, the fin can leave a rim at the proximal end of the cap. Alternatively, a locally enlarged thickness of the cylinder wall of the pen cap, starting at the distal end of the cap and going towards the proximal end, defines the means to prevent the formation of a releasable connection.

The cylinder forming a bore-hole in the pen cap is coaxially arranged within the pen cap and points in the proximal direction. The length, or depth of the bore-hole is such that the bore-hole at least partially encloses the second connector of the cartridge holder when the first connector of the pen cap is connected to the counter connector e.g. when no needle hub is attached to the second connector. Thus by moving the pen cap axially form the first position to the second position, the bore-hole of the pen cap at least partially encloses the second connector.

Preferably, the bore-hole and second connector have a cylindrical shape with an inner diameter of the bore-hole that is above the outer diameter of the second connector. Alternatively, the bore-hole and second connector have an ellipsoidal shape or a rectangular cross section. Important is however that the pen cap or the pen cap forming part of the pen cap assembly and the second connector have matching shapes and are only different in the dimensions as outlined above.

The proximal end of the cylinder forming a bore-hole abuts the second connector when a needle hub is attached to the second connector of the cartridge holder. Preferably, the distal end wall of the needle hub abuts the proximal end of the bore-hole when the cap is axially moved from the first to the second position, and preferably this abutment occurs before the first connector meets the counter connector, e.g. before the pen cap reaches the second position. Thereby the formation of the releasable connection of the pen cap to the injection pen is prevented.

The outer diameter of the needle hub of the injection needle is designed to be above the inner diameter of the bore-hole or the outer diameter of the bore-hole is designed to be below the outer diameter of the needle hub. The pen cap or the pen cap forming part of the pen cap assembly is designed accordingly. As a consequence, the proximal end of the bore-hole abuts the needle hub before the first connector of the pen cap connects to the counter-connector, and the pen cap cannot be moved from the first to the second position.

The pen cap is made from at least one polymeric material using injection molding. Suitable polymers are, for example PBT, POM, PPSU, ABS, PPE, PE, PP, PC, PS, PET, PA and the like. The bore-hole and pen cap are preferably molded as a monolithic part from the same polymeric material in a single injection molding step. Alternatively, the cylinder forming a bore-hole and the pen cap are separately injection molded from identical or different polymeric materials and joined together in a separate step, for example using laser welding or ultrasonic welding. In another alternative, the bore-hole is made from a metal and joined together with the polymeric pen cap during injection molding. The metal insert or bushing is inserted into the mold before the polymeric material is injected. In yet another alternative the pen cap is injection molded first and joined with the metal insert or bushing during a separate step using an adhesive glue or ultrasonic welding or laser welding.

The first connector of the pen cap and the counter connector preferably form a snap-fit connection or a bayonet connection when the pen cap is moved along the longitudinal axis of the injection pen from the first position to the second position. For a snap-fit connection, the connector and/or the counter-connector move axially parallel to the longitudinal axis of the pen before the releasable connection is established. In the alternative of a bayonet connection, the pen cap and the housing or housing part are first axially moved together and subsequently rotated with respect to each other. The formation of the connection can be accompanied by an audible noise or click to indicate to the user that the releasable connection is formed. For releasing the pen cap from the injection pen, the above mentioned steps are reversed, e.g. the pen cap is axially moved away from the housing. An initial reluctance or small force needs to be overcome to close or open the connection.

In the above example, the means for preventing the formation of the releasable connection is described using a bore-hole or bushing. In the following, the means comprising at least one fin on the inside of the cap is described in more detail. The at least one fin protrudes from the cylindrical wall surface of the pen cap towards the central axis of the pen. The at least one fin has a length in the radial direction such that the fin does not abut the second connector of the cartridge holder when the first connector of the pen cap is connected to the counter-connector. The at least one preferably starts at the end-wall of the pen cap and has a length in the longitudinal direction towards the proximal end of the pen cap. The length is chosen such that the at least one fin can interfere with a needle hub once a needle hub is attached to the second connector. The at least one fin abuts the needle hub when a needle hub is attached to the second connector of the cartridge holder thereby preventing the formation of the releasable connection of the pen cap to the injection pen.

The at least one fin is arranged on the inside of the pen cap along a pattern such that when more than one fin is used, the abutment of the fins with the needle hub is distributed over several contact locations. For example if two fins are used, then those two fins are preferably arranged opposite to each other on the inside of the pen cap. For three fins, the fins are preferably arranged on a triangular pattern, for four fins on a cross shaped pattern and for five fins on a pentagonal pattern. The advantage of using a plurality of fins is that the forces upon abutment are distributed, leading to lower contact stresses and therewith materials can be used with a lower stiffness or strength or each fin can be constructed thinner.

The end of the fin can have an arc shaped extension. The arc shaped extension is present on the end of the fin radially pointing towards the central axis of the pen cap. The arrangement is a combination of a fin and a circular shaped bore, where the circle is attached to the radial end of each fin. The circular shaped bore thus can have a continuous circular cross section or a discontinuous cross section. Furthermore not each of the at least one fin on the inside of the cap needs to be equipped with an arc shaped extension, for example only half of the fins can have an arc shaped extension.

The pen cap is made from at least one polymeric material using injection molding and the at least one fin and pen cap are molded as a monolithic part from the same polymeric material such as PBT, POM, PPSU, PC, ABS, PE, PP, PA, PET and the like.

As an alternative, the at least one fin and pen cap are separately injection molded from identical or different polymeric materials and joined together in a separate step, for example using laser welding, or ultrasonic welding. In an another alternative, the at least one fin is made from a metal and joined together with the polymeric pen cap during injection molding of the pen cap or during a separate step.

The pen cap with at least one fin where the first connector and counter-connector form a snap-fit or bayonet connection when the pen cap is moved along the longitudinal axis of the injection pen from the first position to the second position.

An Injection pen or an injection device comprising a pen cap for enclosing a cartridge holder of the injection pen whereby the pen cap comprises:
- a cylindrical wall section or cylindrical wall connecting an opening at the proximal end for inserting the cartridge holder to, or with, a distal end-wall for closing the pen cap; the cylindrical wall section is preferably made from a first material,
- whereby the pen cap is characterized by that the distal end wall comprises a second material reinforcing the distal end wall and the reinforcing material prevents needle stitching through the end wall.
The pen cap has the advantage that it prevents stitching of the needle through the distal end wall which might get unnoticed by the user and therewith prevents injuries for the user. A pen cap for an injection device whereby the pen cap comprises the cylindrical wall section and end wall, the cylindrical wall section is preferably made of the first material and the end wall comprises the second material preventing needle stitching through the end wall.

The first material is preferably a plastic material and preferably a commodity plastic such as a polyolefin (PE, PP, HDPE), or polystyrene (PS) or Polyvinylchloride (PVC). These plastics are cost efficient and reduce the material costs for the cap.

The second material of the pen cap at least partially covers the end wall of the pen cap, preferably in the central area of the pen cap. In this option, the end wall of the pen cap is made at least of the first and the second material whereby the second material reinforces the pen cap preventing needle stitching. Alternatively, the distal end wall is completely made of the second material without using the first material. Thus the end wall is, for example, inserted as a separate part closing the cylindrical wall section of the pen cap. In yet another alternative the end wall is at least partially made from the second material.

The end wall of the pen cap comprises the first material or the second material, or the end wall comprises the first material and the second material. The second material can be located on the inside surface of the pen cap at the surface of the end wall facing the opening of the pen cap. Alternatively the second material is located on the outside surface of the distal end wall being furthest away from the opening of the pen cap.

The second material of the pen cap for the injection pen can be a fiber reinforced or particulate filled plastic material. The second plastic material can be selected from a commodity plastic (PE, PP, PS, PVC) which is reinforced by hard particles present in the plastic. The hard particles can be glass particles, aluminiumoxide particles, sand (silicium-dioxide or quartz-sand), zirconiumdioxide particles or siliciumcarbide particles or corundum particles. Alternatively, the second material is a fibre reinforced plastic comprising glass fibres or carbon fibres.

In yet another alternative, the second material reinforcing the pen cap is a metal such as iron, stainless steel, copper, brass or the like.

In yet another alternative, the second material is a second plastic material, e.g. without any particulate orfibre filing. The second material preferably has a modulus above the modulus of the first material. The modulus is used here for the stiffness of the material and can be a tensile, compression or shear modulus depending on the materials test used to define the stiffness property of the material. Preferably a tensile modulus value tested at room temperature according to a certified standard such as ASTM F638 is used to compare stiffness values between the first and second material. The second material is preferably selected from the group of engineering thermoplastics such as Polybutyleneterphthalate (PBT), Polyethyleneterphthalate (PET) or Polyacetal POM. Alternatively the second material is selected from the group of high performance thermoplastics such as Polyether etherketone (PEEK), PPSU, PEI (Poyetherimide), Polyphenylenes (PPE, PPO, PPS) or polysulfones (PSU). In another example, the second material is a thermosetting material such as an epoxy, polyester, phenolic resin or a polyurethane. These materials can be used as such or as a blend of two polymeric materials or are filled with the above mentioned particles or fibres.

The second plastic material preferably has a modulus at least twice the modulus of the first plastic material. For example the first material can be a relatively low modulus polypropylene, having a young's or E-modulus (tensile) of about 1 GPa. The second material can be a non-filled material such as a polyimide with a modulus of 3 GPa or PEEK (4 GPa). Alternatively the second material is a glass fiber reinforced material having moduli above 5 GPa. For example a glass fibre reinforced polyester (thermoset) or glass fibre reinforced polypropylene (thermoplastic) can be used. The important value is the relative value of the stiffness parameter chosen, thus that the second material has a stiffness at least twice the stiffness of the first material. The higher stiffness of the second material ensures that the needle cannot penetrate the second material. The needle stitching risk also depends on the layer thickness of the first and second materials respectively. The layer thickness of the second material is below 5mm, preferably below 3 mm and more preferably between 0.5 and 2 mm.

The second material, whether present on the inside or outside of the pen cap can be part of, or forms an attachment means, for example a clip of the pen cap. The attachment means can be made of the second material only, but can also comprise the first material or even a third material. This has the advantage that the functionality of an attachment means for the pen cap is combined with the needle stitch prevention.

As described above, pen caps are disclosed having a means to prevent attachment of the pen cap when a needle is attached to the pen cap. To ensure that all needles, for example having lengths between 4 and 20 mm, are enclosed within the cap without interference with the end wall of the cap, the length of the cap must be adjusted to the longest needle provided. In this example, the length of the cap needs to be adjusted to the 20 mm long needle. In practice, most needles used for subcutaneous self-injection, e.g. over 90% of those needles are shorter than 8 mm. Thus for most needles the pen caps are designed too long to encompass the whole range of needle lengths. In order to provide shorter pen caps having a means to prevent attachment of the pen cap which are designed for shorter needles, e.g. for 90% of the users in the above mentioned example, it is required to implement a stitch prevention of the end wall for the limited number of users using needle lengths above 8 mm. Therefore, the injection pen comprising the pen cap further comprises a housing and a cartridge holder which is connectable to the distal end of the housing for holding a cartridge containing a medication. The cartridge holder has a second connector present at the distal end for connecting a needle hub of an injection needle to the cartridge holder. Furthermore, the pen cap further comprises a first connector made of the first material which is located at the proximal end of the opening of the pen cap for connecting to a counter connector present at the distal end of the housing. The first connector is arranged to form a releasable connection of the pen cap to the housing when the pen cap is moved along the longitudinal axis of the injection pen from a first position where the first connector and counter connector are not connected, to a second position establishing the releasable connection between the first connector and the counter connector thereby fully enclosing the cartridge holder, wherein the pen cap has a means for preventing the formation of the releasable connection of the pen cap when the needle hub of the injection needle is connected to the second connector of the cartridge holder and the means is located on the inside surface of the end wall facing the opening. The end wall of this pen cap having the means for preventing attachment is reinforced with the second material preventing needle stitching through the end wall. The second material can be an engineering plastic, high performance plastic as described above with or without a filler. Therewith, a shorter pen cap with the means for preventing attachment can be designed without the risk of needle stitching when the user uses a longer, for example 12 or 20 mm needle.

The injection pen comprising a pen cap wherein the means for preventing the formation of a releasable connection is a cylindrically shaped protrusion forming a bore hole, the protrusion points towards the opening of the pen cap. The cylindrically shaped protrusion forming a bore hole is preferably coaxially arranged in the pen cap and has a length and inner diameter such that the bore hole at least partially encloses the second connector of the cartridge holder when the pen cap is connected to the housing without a needle attached. The proximal end of the cylindrically shaped protrusion forming a bore hole abuts the needle hub of the injection needle when a needle hub is attached to the second connector of the cartridge holder thereby preventing the formation of the releasable connection of the pen cap to the injection pen. Alternatively the means for preventing a releasable connection is at least one fin protruding from the cylindrical wall section or a locally enlarged wall thickness of the cylindrical wall section, said fins or enlarged wall section forming a cavity that functionally behaves like the bore described above.

A method for manufacturing a pen cap for use in the injection pen according to the previous examples is disclosed, said method comprising the steps of:
- Providing the second material in an injection mold
- Injection molding the first material into the mold thereby encapsulating the second material.
The second material can be an inlay or a clip for the pen made, for example from a metal. The first material is molded around the first material thereby forming a bond between the two materials. The bonding between the two materials can be based on a mechanical interaction or interlocking of the two materials. For example the inlay of the second material may comprise holes, protrusions or roughened sections that are filled or enclosed during injection molding of the first material. The cut-outs can be punched out of a layer of the second material. The means for preventing a releasable connection can be also present in the mold before injecting the first material, thus as an example both the second material and a cylindrical metal insert can be inserted in the mold and are overmolded by the first material. The second material can be inserted into the mold as a separate part such as a clip or a layer of reinforced plastic. Alternatively the second material is first injection molded into the mold before molding the first material.

### Legends to Figures

Figure 1: Longitudinal section of a pen cap mounted on an injection pen of the prior art having no means to prevent stitching of the cap and no means to prevent mounting of the cap when a needle hub is attached to the cartridge holder,
Figure 1a: Axial view (bottom) on the pen cap of the prior art,
Figure 1b: 3-Dimensional view of the pen cap of the prior art,
Figure 2: Longitudinal section of a pen cap mounted on an injection pen, the pen cap having an increased distal wall thickness and means to deflect the needle tip of the injection needle,
Figure 3: Longitudinal section of a pen cap mounted onto an injection device, the pen cap having a distal bore-hole preventing the formation of the releasable connection of the pen cap to the injection device,
Figure 3a: Bottom view of the pen cap having a bore-hole,
Figure 3b: 3-Dimensional view of the pen cap having the bore-hole,
Figure 3c: Longitudinal section of the pen cap having the bore-hole, the pen cap is mounted onto the injection pen and the bore hole encloses the second connector of the cartridge holder,
Figure 4: Longitudinal section of a pen cap having one fine on the inside of the cap. The pen cap is mounted onto the injection device,
Figure 4a: Bottom view of the pen cap having one fin,
Figure 5: Longitudinal section of a pen cap having two fins oppositely arranged within the pen cap,
Figure 5a: Bottom view of the pen cap having two fins,
Figure 6: Longitudinal section of a pen cap mounted onto the injection device, the pen cap having 4 protruding fins on the inside of the cap,
Figure 6a: Bottom view of the pen cap having 4 fins,
Figure 7: Longitudinal section of the pen cap having 4 fins, a needle hub is attached to the cartridge holder which prevents the formation of the releasable connection of the pen cap to the injection pen,
Figure 8: Longitudinal section of a pen cap for an injection pen, the pen cap having a bore-hole as a metal insert in the distal end of the pen cap,
Figure 8a: Longitudinal section of a pen cap having a metal insert. The formation of the releasable connection of the pen cap to the injection pen is prevented since a needle hub is attached to the cartridge holder,
Figure 9: Bottom view of a pen cap having both fins protruding towards the centre of the cap with arc shaped extensions attached to the fins,
Figure 9a: 3-D view of the pen cap having both fins and arc shaped extensions,
Figure 10a: Pen cap with a reinforced end wall for preventing stitching through the end-wall,
Figure 10b: Longitudinal section of the pen cap according to Figure 10a, showing the reinforcement on the outside of the cap,
Figure 11: Longitudinal section of a pen cap having the clip made of a reinforced material; the reinforcement of the end wall is present on the inside of the pen cap,
Figure 12a: Longitudinal section of a pen cap having the reinforced material as an overlay of the end wall and the clip,
Figure 12b: Pen cap according to Figure 12a,
Figure 13: Pen cap with a reinforced material on the outside of the cap, the reinforced material is at least partially attached to the pen cap (first material) using cut-outs in the reinforced material,
Figure 14a: Longitudinal section of pen cap whereby the second material is injection molded first into the mold in the end-wall section, followed by molding the first material of the pen cap,
Figure 14b: Pen cap according to Figure 14a,

### Detailed description

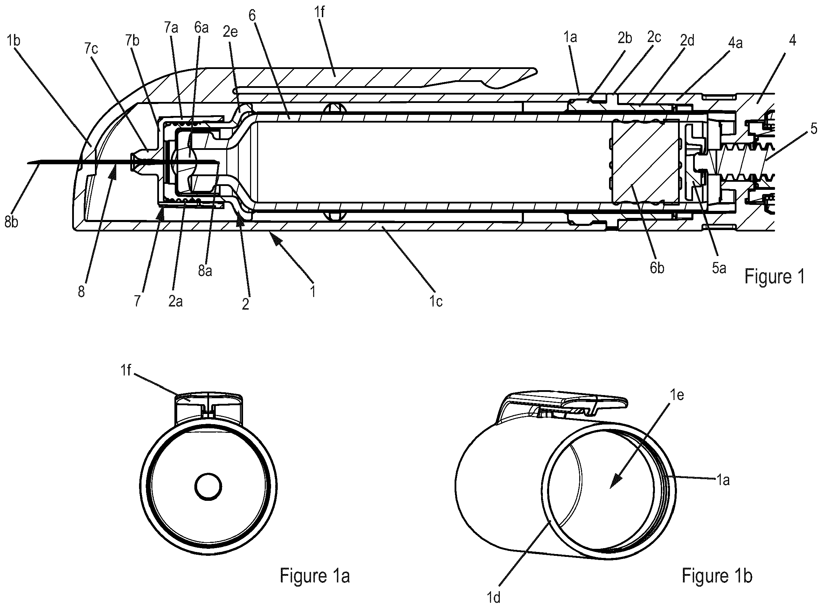

In Figure 1, a pen cap (1) is presented that is attached to an injection pen. Only a distal part of the injection pen is shown comprising a housing (4), a piston rod (5) having a flange (5a) at the distal end of the piston rod (5). The dose setting mechanism and the coupling mechanisms between the dose setting and the dose delivery mechanism are not shown. The pen cap or pen cap assembly can be used for a wide diversity of drive mechanisms, including but not limited to spring driven pens, manual activated pens, electromechanically driven pens, gas driven pens and the like. The injection pen furthermore comprises a cartridge holder (2) which is connected to the distal end of the housing (4). The cartridge holder (2) has an opening on the proximal end for receiving a cartridge (6). The cartridge holder (2) has a cartridge holder connector (2d) at the distal end which is designed to connect to a counter connector (4a) present at the distal end of the housing (4). The connectors between the housing and the cartridge holder can be, for example, a bayonet coupling. The cartridge holder connector (2d) can be shaped as a protrusion that matches a guide slot present on the inside of the housing (4). The guide slot represents the counter connector (4a) and can guide the cartridge holder (2) first axially which is then followed by a rotational movement. The guide slot can thus be designed such that the connection requires for axial and rotational movements and combinations thereof.

The cartridge holder (2) is tubular shaped for receiving the cartridge (6). At the distal end of the cartridge holder (6), the diameter is reduced to form a shoulder section (2e) and a second connector (2a) is present at the distal end. The second connector (2a) is designed for connecting to a needle hub (7) which holds a needle (8). The needle hub (7) is cylindrically shaped with a wall surface (7a) having an inside surface suited for connecting to the second connector (2a) of the cartridge holder (2). The connecting surfaces can be designed as a screw thread, or multiple circumferential ridges and the like. The cylinder wall (7a) of the needle hub (7) can comprise flexible arms to flex over the ridges or threads of the second connector (2a). Such connecting surfaces are known in the art and called, for example, a clickfine needle connection. The needle hub (7) has a central unit or needle holder unit (7c) for holding a hollow steel needle or cannula (8). The needle (8) is coaxially arranged within the needle hub (7) and has two sharp needle ends (8a, 8b). The distal needle end (8b) is used for penetrating the skin of the patient and the proximal needle end (8a) penetrates a septum (6a) which is present at the distal end of the cartridge (6). Once a needle hub (7) is attached to the second connector (2a) of the cartridge holder (2), then the septum (6a) of the cartridge (6) is pierced to form a connection between the inside of the cartridge (6) and the outside. Once the drive mechanism of the injection pen advances the piston rod (5), the medication will be expelled from the cartridge (6) via the needle (8) to the patient.

The injection pen is closed off by a pen cap (1). The pen cap (1) comprises an end wall (1b) and a cylinder wall surface or wall section (1c). When the pen cap (1) is mounted onto the housing (4) or onto the cartridge holder (2), the cartridge holder containing the cartridge is covered by the pen cap to prevent damage to the cartridge holder (2) having the second connector (2a) or to the cartridge (6). The pen cap (1) may have a clip (1f) on the outside of the cylinder wall surface (1c) which ensures that the injection pen can be carried in, and attached to, for example a pocket. The pen cap (1) has a proximal opening (1e, Figure 1b) and the user slides the opening (1e) of the pen cap (1) over the cartridge holder (2) towards the housing (4). The pen cap (1) has a first connector (1a) located at the proximal end of the pen cap. In the example shown, the first connector (1 a) is shaped as a circumferential recess on the inside at the proximal rim of the pen cap (1), see Figures 1 and 1b. The pen cap (1) is axially moved from a first position whereby the pen cap is aligned with and surrounds the cartridge holder, but still does not fully enclose the holder, to a second position then the connection between the first connector (1a) and the counter connector (2b) is closed. During the movement from the first to the second position, the first connector (1a) and/or the counter connector (2b) radially flex over each other to form the connection which can be accompanied by an audible clicking noise. The connection established is called a snap-fit connection. The counter connector (2b) in the example shown in Figure 1 is located at the proximal section of the cartridge holder (2) but for the skilled person the counter connector can also be located at the housing (4), or a housing part which is attachable to the housing (4).

In the example shown in Figure 1, the user has first removed the pen cap (1) from the injection pen, attached a needle hub (7) to the cartridge holder and subsequently removed the needle cover and needle tip cap (both are not shown in Figure 1). Then the user sets the desired dose, inserts the distal end (8b) of the needle (8) into the skin and performs the injection. The needle should be used normally only once, and it is recommended to put the needle cover again over the distal end of the needle and remove the needle hub (7) with the needle (8) from the injection pen. Finally, the user should re-attach the pen cap (1) to the device. If a user forgets to remove the needle hub (7) including the needle (8), then the distal end (8b) of the needle can pierce the end wall (1b) of the pen cap (1) when the pen cap is re-attached to the injection pen (Figure 1). For this to happen, several requirements should be met; the head space for the needle in the pen cap (1) after connecting the first connector (1a) to the counter connector (2b) should be restricted, the length of the needle (8) should be long enough to abut the end wall (1b) of the pen cap (1) when the pen cap is moved from the first to the second position and the material of the end-wall is too weak to prevent stitching through the end-wall. The puncturing of the end wall (1b) of the pen cap represents a risk to the user which should be avoided as well as the risk of re-using single use needles multiple times.

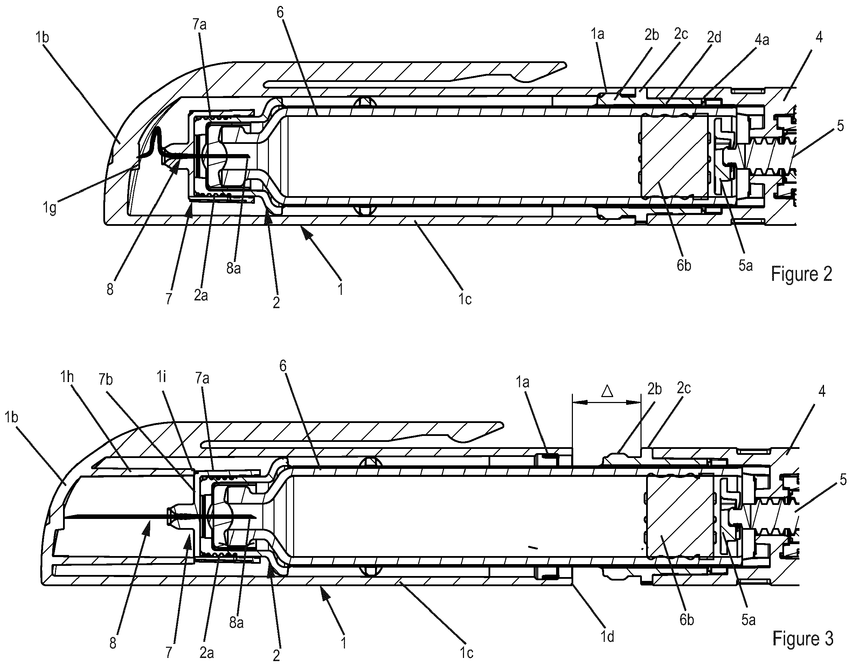

In Figure 2a first embodiment of a pen cap for preventing re-using single-use needles is shown. The pen cap (1) has a thicker end wall (1b) which prevents that the wall can be pierced by the distal end (8b) of the needle (8). The pen cap is made from a first material. Additionally, the end-wall of the pen cap (1) can have an additional protrusion (1g), preferably cone shaped, in the centre at the distal end wall (1b) of the pen cap (1). The thicker end wall (1b) and/or the cone shaped protrusion (1g) prevent the piercing through the cap. Additionally, the cone shaped protrusion (1g) can deflect the needle (8) such that the needle cannot be used anymore. The destruction of the single use needle prevents re-use of the needle and the user is reminded to the single use-purpose, should he or she forget once to remove the needle hub (7) before reattaching the pen cap (1). The deflection means can be a cone shaped protrusion but can also be ridges arranged on a cross or triangular shaped pattern or can be a combination of a conus and ridges.

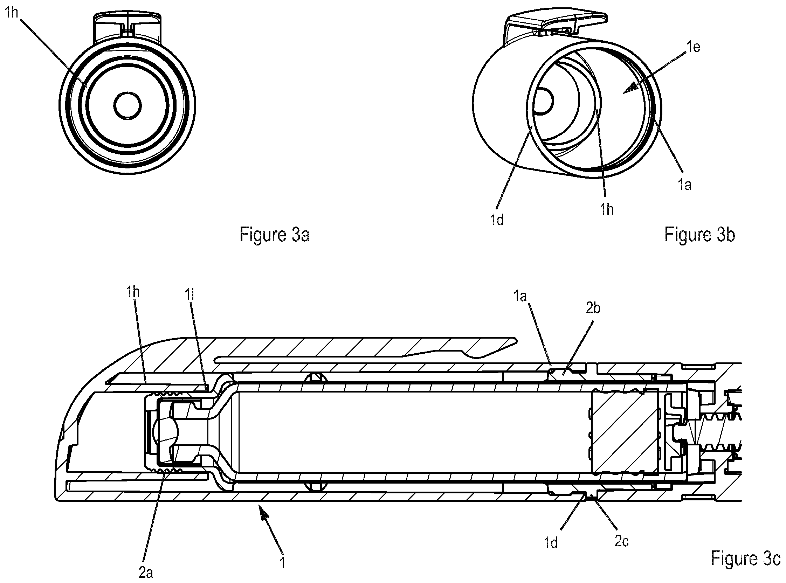

In Figure 3, a second embodiment is shown of the pen cap (1) or of a pen cap (1) forming part of a pen cap assembly. The pen cap (1) has a cylindrical insert forming a bore-hole (1h) which is attached to the distal end wall (1b) of the pen cap (1). The bore-hole (1h) is coaxially arranged within the pen cap (Figure 3a). In Figure 3, the pen cap (1) is in the second position, a needle hub (7) with a needle (8) is attached to the cartridge holder, e.g. a needle hub (7) is attached to the second connector (2a). The user attempts to attach the pen cap (1) and axially shifts the pen cap (1) over the cartridge holder (2). By moving the pen cap (1), the proximal end (1i) of cylinder forming the bore-hole (1h) abuts the end-wall (7b) of the needle hub (7) before the first connector (1a) of the pen cap (1) can connect to the counter connector (2b) present at the cartridge holder (2). A gap indicated with Δ is left between the housing (4) and the pen cap (1) and the user can still see part of the cartridge holder (2) or the cartridge (6). The bore-hole (1 h) thus prevents re-attachment of the pen cap (1) to the injection pen when a needle hub (7) is still attached to the cartridge holder (2). Furthermore, the bore-hole (1 h) prevents that the end-wall (1 b) gets stitched by the needle (8).

In Figure 3c, the pen cap (1) is attached to the injection pen without a needle hub (7) present at the cartridge holder (2). The connection between the first connector (1a) and the counter connector (2b) has been established. The user shifted the pen cap (1) over the cartridge holder (2) from the first to the second position until the proximal rim (1d) of the pen cap (1) abuts the axial stop (2c) of the cartridge holder and the snap-fit connection (1a, 2b) has been formed, preferably accompanied by an audible click. During the axial movement of the pen cap (1), the cylinder forming the bore-hole (1 h) shifts at least partially over the second connector (2a) of the cartridge holder (2).

The pen cap (1) and the cartridge holder (2) are designed such that the inner dimensions of the bore-hole (1h) fit over the outer dimensions of the second connector (2a) of the cartridge holder. In the second embodiment, the bore-hole (1 h) and the second connector (2a) have a circular cross section (see Figure 3a), but also other shapes can be envisaged for the cross section. The pen cap (1) and the cartridge holder (2) thus may form a pen cap assembly whereby the dimensions in terms of axial length of the pen cap, positioning of the first connector, shape and length of the bore hole and the shape and length of the second connector are adjusted to each other. Such a specially designed pen cap assemblies may also prevent that a pen cap and/or a cartridge holder from one device is used for another device normally requiring a different pen cap.

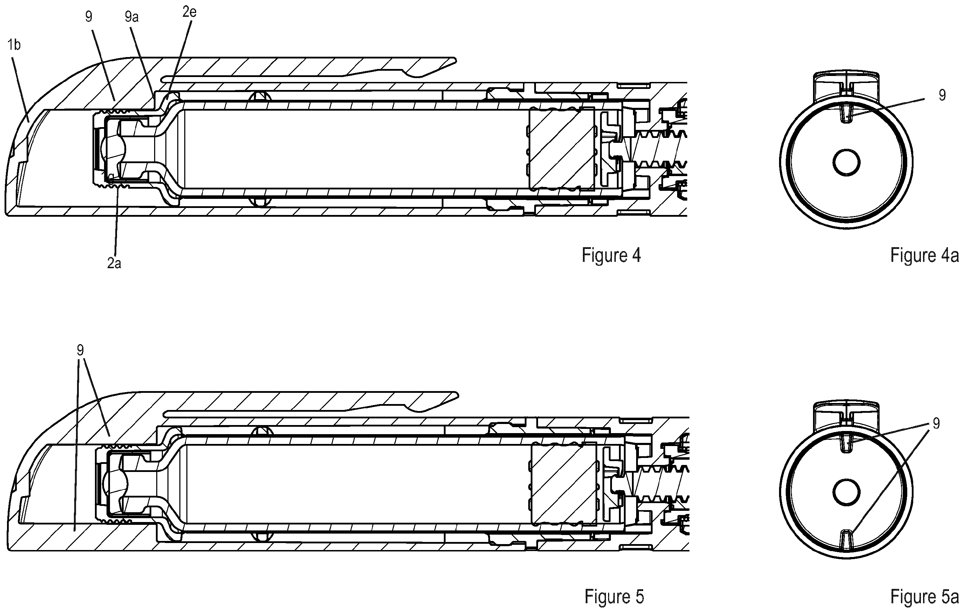

In Figure 4 a third embodiment of the pen cap (1) is shown. The pen cap (1) has one fin (9) that starts at the distal end-wall (1 b) of the pen cap (1). In the radial direction, the fin (9) has a length such that the fin does not interfere with the second connector (2a) when the pen cap is attached to the injection device. The fin (9) has a length pointing in the proximal direction such that is does not interfere with the shoulder (2e) of the cartridge holder (2). Once a needle hub (7) is attached to the cartridge holder, the proximal end (9a) of the fin (9) will abut the needle hub before the pen cap can be attached to the injection pen. In that case, the pen cap cannot be moved from the first to the second position.

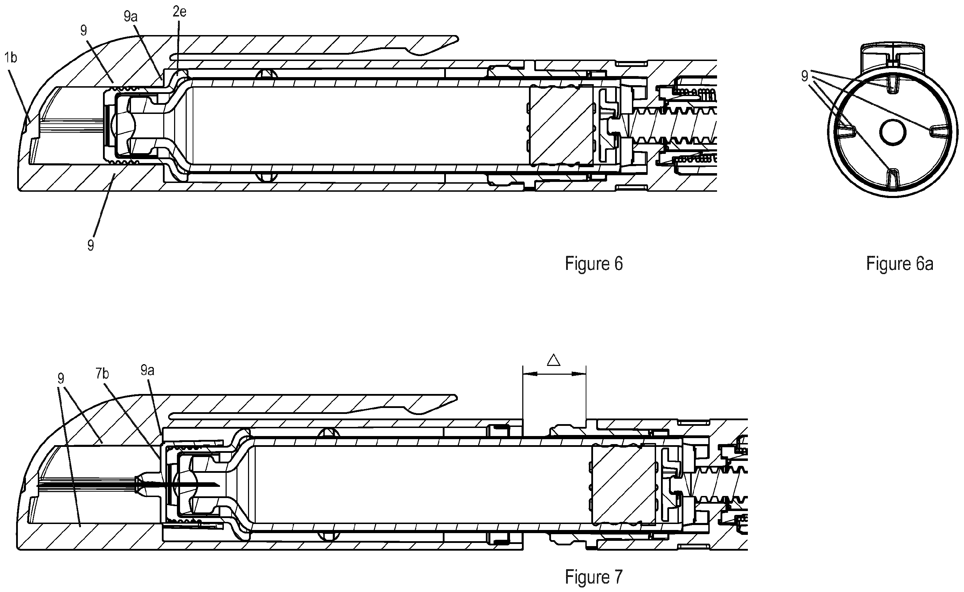

In Figures 5 and 6 the fourth and fifth embodiments are shown having two respectively four fins present on the inside of the pen cap (1). The fins are oppositely arranged (Figure 5a) or in a cross shaped pattern (Figure 6a) and the functioning is essentially the same as for the third embodiment. The two respectively four fins can also be arranged on different patterns.

In Figure 7, the prevention of the formation of the connection between the pen cap (1) and the injection device is shown for the fourth and fifth embodiment. The cross section shows that the proximal ends (9a) of the fins (9) abut the end-wall (7b) of the needle hub (7) before the connection between the first connector (1a) of the pen cap and the counter connector (2b) of the cartridge holder (2) can be established, thus leaving a Δ gap between the pen cap (1) and the housing (4).

In the first to fifth embodiments, the means to prevent the formation of the connection between the pen cap (1) and the injection pen are directly injection moulded together with the pen cap (1), e.g., the at least one fin (9) and the cylinder forming the bore-hole (1h) are made in a single step together with the pen cap and preferably made from the same material. However, the mechanical demands on the at least one fin (9) or the bore-hole (1 h) might be higher since a user may attempt to reattach the pen cap (1) to the device using excessive forces. Therefore, the at least one fin (9) or bore-hole (1 h) can also be made from a different material, for example from a high strength/stiffness material such as a fibre reinforced polymeric material. The at least one fin (9) or bore-hole (1 h) are produced in a separate step and injection molded as an inlay together with the molding of the pen cap. Alternatively, both parts are first produced and joined together in a separate step using, for example, laser welding or ultra-sonic welding or adhesive glueing techniques and the like.

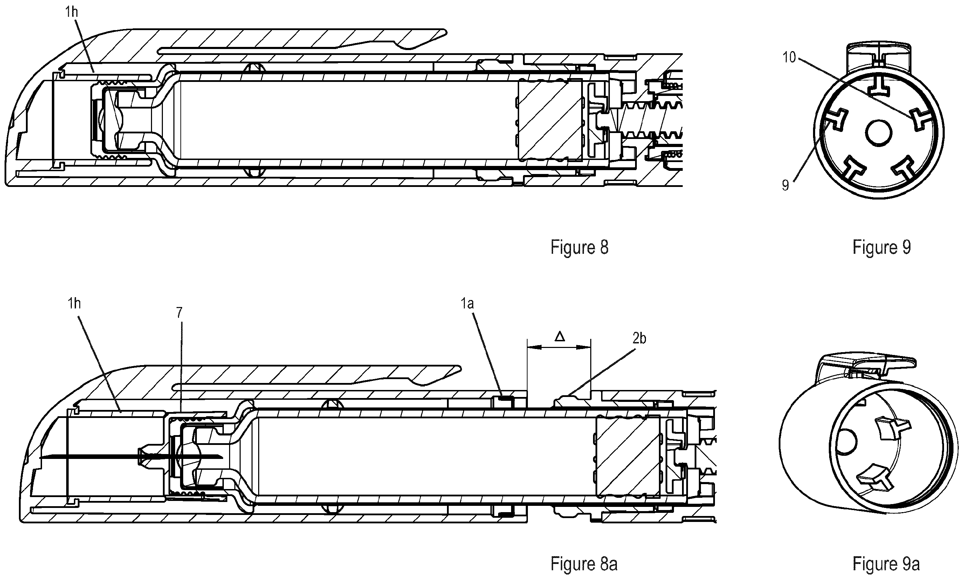

In Figure 8, a sixth embodiment is shown whereby the bore-hole (1h) of the pen cap is a metal insert that is inserted as an inlay into the mold prior to injection molding the pen cap (1). In Figure 8a it is illustrated how the bore-hole (1 h) prevents the formation of the releasable connection between the first connector of the pen cap (1) and the counter connector (2b) at the proximal section of the cartridge holder (2) when a needle hub (7) is attached to the cartridge holder (2).

In Figures 9 and 9a a sixth embodiment is presented which forms a partial combination of a bore-hole and a fin. The at least one fin (9) has an arc shaped extension (10) attached to the end of the fin (9) that points in the radial direction towards the center of the pen cap (1). Depending on the length of the arc shaped extension (10), the gaps between the arc shaped extensions (10) can be varied. Once the arc shaped extensions abut each other, the means to prevent cap attachment is a combination of a fin and a cylinder forming a bore-hole. In the sixth embodiment, five fins protrude from the wall of the pen cap (1). For the skilled person, the arc shaped extensions can also be combined with any of the other embodiments described above having one, two or four fins.

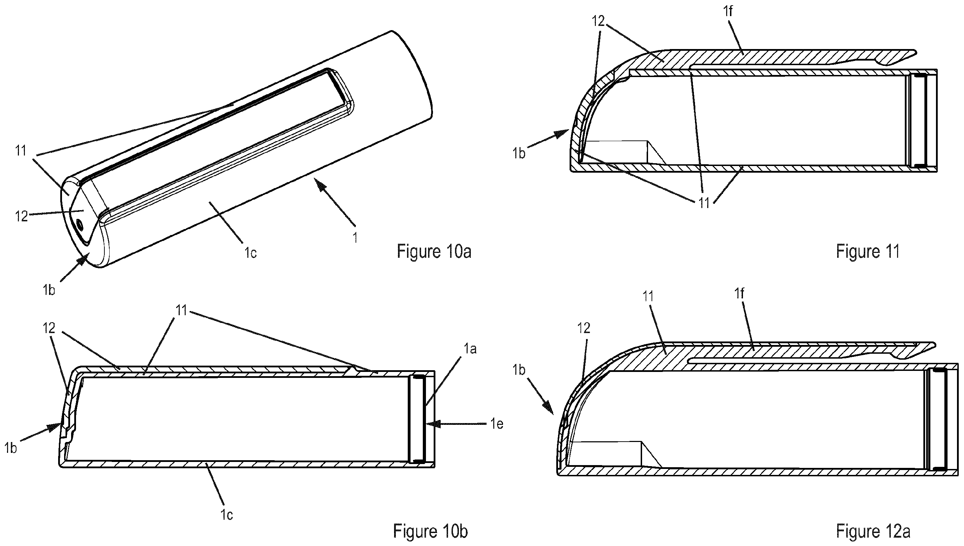

Figures 10a and 10b display a pen cap (1) for an injection pen according to a seventh embodiment. The pen cap has a stitch prevention as part of the end wall (1b). The pen cap (1) comprises a cylindrical wall section (1c) made of a first material (11), preferably a plastic material such as polypropylene. Polypropylene is a cost efficient commodity plastic having a relatively low stiffness (tensile modulus of about 1 GPa) and low stitch resistance. In order to prevent cap stitching as shown in Figure 1, a relatively thick polypropylene end wall needs to be chosen, preferably above 2 mm, more preferably above 3mm, even more preferably above 4 mm. Alternatively, a second material (12) is present on the outside of the pen cap, whereby the reinforced material (12) is at least present in the central area of the end-wall which can be penetrated by the distal end (8b) of a needle (8). The second material preferably is a fiber reinforced plastic, for example a polypropylene or polybutylene terephthalate polymer reinforced with glass fibers. Alternatively the second material is a sheet of stainless steel or made of a non-filled plastic material such as PPSU or PEEK. The latter polymers having a tensile modulus above 3 GPa (tested room temperature) and are stiff plastic materials which, as such, provide a stitch resistance without the need of a filler.

An eight embodiment is presented in Figure 11. The pen cap for the injection pen is made of the same first material (11) as for the seventh embodiment. The second material is part of clip (1f). Clip (1f) is designed as one part and arranged in the pen cap such that the second material (12) is on the inside of the cap facing the opening. The second material is preferably made from a stiffer material to prevent needle stitching through the end wall (1b). The integration of the stiffer material in the clip has the additional advantage of having a more reliable (less bending) clip (1f) as attachment means for the pen cap.

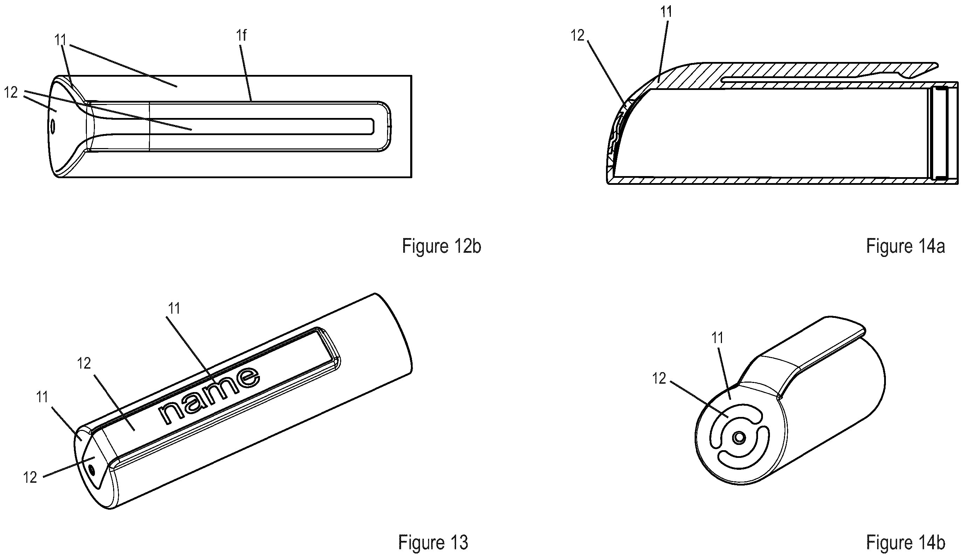

In Figures 12a and 12b a ninth embodiment is presented of a pen cap. The second material (12) at least partially covers the outside surface of the end wall (1b). In this embodiment, the clip (1f) is partially covered with the second material (12) to stiffen the clip (1f). The second material can be selected from the same group as the seventh embodiment.

Figure 13 shows a pen cap according to a tenth embodiment. The second material (12) is provided as an inlay having cut-outs (punched parts) where the first material can penetrate to the surface, thereby improving the fixation between the first material (11) and the second material (12). As an example, the first material (11) is made from a plastic material such as PP, PE or PBT and the second material is a sheet of metal having the cut-outs, in the example shaped as the word "name", however any other form can be envisaged for the cut-outs.

In Figures 14a and 14b an eleventh embodiment is presented of the cap. The second material (12) is present both at the inside surface of the end wall (1b) as well as on the outside. The first material (11) is molded around the second material using the following method: First the second material (12), for example a glass fibre reinforced plastic, is molded into an injection mold and thereafter the second material (12) is molded on top of, and around the first material. As an alternative, the second material (12) is provided as an inlay, for example a metal inlay, and the first material (11) is subsequently molded around the inlay.

The means for preventing the formation of a releasable connection of the pen cap as presented in any of the embodiment one to six can be combined with the needle stitch prevention as shown in embodiments seven to eleven. For example, the bore hole (1 h) as presented in Figure 3 or the at least one fin (9) presented in Figures 4 to 9 can be combined with a reinforcement of the end wall (1 b) of the pen cap as disclosed in Figures 10 to 14.

### Part annotation

- 1: Pen cap
- 1a: First connector
- 1b: End wall pen cap, distal end-wall
- 1c: Cylinder wall pen cap, cylindrical wall section
- 1d: Proximal rim
- 1e: Proximal opening pen cap
- 1f: Clip
- 1g: Protrusion
- 1h: Bore-hole, cylinder forming a bore hole
- 1i: Proximal end bore hole
- 2: Cartridge holder
- 2a: Second connector
- 2b: Counter connector
- 2c: Axial stop
- 2d: Cartridge holder connector
- 2e: Shoulder
- 4: Housing
- 4a: Counter connector cartridge holder
- 1a,2b: Snap fit connection
- 5: Piston rod
- 5a: Flange
- 6: Cartridge
- 6a: Septum
- 6b: Plug
- 7: Needle hub
- 7a: Cylinder wall needle
- 7b: End wall needle hub
- 7c: Needle holder
- 8: Needle
- 8a: Proximal needle end
- 8b: Distal needle end
- 9: Fin
- 9a: Proximal end
- 10: Arc shaped extension
- 11: First material
- 12: Second material

## Claims

1. Injection pen comprising a pen cap (1) for enclosing a cartridge holder (2) of the injection pen the pen cap comprising:
a cylindrical wall section (1c) connecting an opening (1e) at the proximal end for inserting the cartridge holder (2) to a distal end wall (1 b) for closing the pen cap,
the cylindrical wall section (1c) is made from a first material (11),
**characterized in that** the end wall comprises a second material (12) reinforcing the end wall (1b) and preventing needle stitching through the distal end wall (1b).

2. The injection pen comprising a pen cap (1) according to claim 1 wherein the first material (11) is a plastic material.

3. The injection pen comprising a pen cap (1) according to claims 1 or 2 wherein the second material (12) at least partially covers or at least partially builds the distal end-wall (1 b) of the pen cap.

4. The injection pen comprising a pen cap (1) according to claims 1 to 3 wherein the distal end wall (1b) comprises the first material (11).

5. The injection pen comprising a pen cap (1) according to claims 1 to 4 wherein the second material (12) is located on the inside surface of the distal end wall (1b) facing the opening (1e).

6. The injection pen comprising a pen cap (1) according to claims 1 to 4 wherein the second material (12) is located on the outside surface of the distal end wall (1b) being furthest away from the opening (1e).

7. The injection pen comprising a pen cap (1) according to any of the previous claims wherein the second material (12) is a fiber reinforced or particulate filled plastic.

8. The injection pen comprising a pen cap (1) according to claims 1 to 6 wherein the second material (12) is a metal.

9. The injection pen comprising a pen cap (1) according to claims 1 to 6 wherein the second material (12) is a second plastic material having a modulus above the modulus of the first material (11).

10. The injection pen comprising a pen cap (1) according to claim 9 wherein the second plastic material (12) has a modulus at least twice the modulus of the first plastic material (11).

11. The injection pen comprising a pen cap (1) according to any of the previous claims whereby the second material (12) is part of, or forms an attachment clip (1f) for the injection pen.

12. The injection pen comprising a pen cap (1) according to any of the previous claims further comprising A housing (4),
a cartridge holder (2) connectable to the distal end of the housing (4) for holding a cartridge (6) containing a medication, the cartridge holder (2) having a second connector (2a) present at the distal end for connecting a needle hub (7) of an injection needle to the cartridge holder (6),
the pen cap (1) further comprising a first connector (1 a) made of the first material (11) which is located at the proximal end for connecting to a counter connector (2b) present at the distal end of the housing (4) or at the proximal end of the cartridge holder (2),
said first connector (1a) is arranged to form a releasable connection (1a,2b) of the pen cap (1) to the housing (4) when the pen cap (1) is moved along the longitudinal axis of the injection pen from a first position where the first connector (1a) and counter connector (2b) are not connected, to a second position establishing the releasable connection (1a,2b) between the first connector (1a) and the counter connector (2b) thereby fully enclosing the cartridge holder (2), wherein the pen cap (1) has a means (1 h, 9,10) for preventing the formation of the releasable connection of the pen cap when the needle hub (7) of the injection needle is connected to the second connector (2a) of the cartridge holder and the means (1h, 9,10) is located on the inside surface of the distal end wall (1b) facing the opening (1e).

13. The injection pen comprising a pen cap (1) according to claim 12, wherein the means (1h, 9,10) for preventing the formation of a releasable connection (1a, 2b) is cylindrically shaped protrusion forming a bore hole (1 h), the protrusion pointing towards the opening (1e) of the pen cap (1) or at least one fin (9) protruding from the cylindrical wall section (1 c) or a locally enlarged wall thickness of the cylindrical wall section (1c).

14. The injection pen comprising a pen cap (1) according to claim 13 wherein the cylindrically shaped protrusion forming a bore hole (1 h) is coaxially arranged in the pen cap and having a length and inner diameter such that the bore hole at least partially encloses the second connector (2a) of the cartridge holder (2) when the pen cap (1) is connected to the housing (4).

15. The injection pen comprising a pen cap (1) according to claim 14 wherein the proximal end (1i) of the cylindrically shaped protrusion forming a bore hole (1 h) abuts the needle hub (7) of the injection needle when a needle hub (7) is attached to the second connector (2a) of the cartridge holder (2) thereby preventing the formation of the releasable connection (1a, 2b) of the pen cap to the injection pen.

16. A method for manufacturing a pen cap (1) for use in the injection pen according to claims 1 to 10 comprising the steps of:
- Providing the second material (12) in an injection mold
- Injection molding the first material (11) into the mold thereby at least partially encapsulating the second material (12).

17. The method for manufacturing a pen cap (1) according to claim 16 wherein the second material (12) is provided by injection molding the second material (12).
